(19) Europäisches Patentamt
European Patent Office
Office européen des brevets

(11) Publication number: **0 026 542**
**B1**

(12) **EUROPEAN PATENT SPECIFICATION**

(45) Date of publication of patent specification: **15.12.82**

(51) Int. Cl.³: **C 07 C 120/00,**
C 07 C 121/66,
C 07 C 121/75

(21) Application number: **80200905.0**

(22) Date of filing: **26.09.80**

(54) **Preparation of esters containing an alpha cyano group in the alcohol portion of the ester.**

(30) Priority: **27.09.79 US 79622**
**27.09.79 US 79635**
**27.09.79 US 79610**
**06.06.80 US 156958**
**13.06.80 US 159085**
**13.06.80 US 159338**

(43) Date of publication of application:
**08.04.81 Bulletin 81/14**

(45) Publication of the grant of the patent:
**15.12.82 Bulletin 82/50**

(84) Designated Contracting States:
**CH DE FR GB IT LI NL**

(56) References cited:
EP - A - 0 002 288
DE - A - 2 825 197

E. V. DEHMLOW et al. "Phase Transfer Catalysis" 1980, VERLAG CHEMIE, Weinheim * pages 46 to 52 *

(73) Proprietor: **FMC Corporation**
**2000 Market Street**
**Philadelphia Pennsylvania 19103 (US)**

(72) Inventor: **Baum, Jonathan Sheffield**
**148 South Main Street**
**Pennington New Jersey 08534 (US)**

(74) Representative: **Kooy, Leendert Willem et al,**
**Dr. Kuyperstraat 6**
**NL-2514 BB Den Haag (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



**EP 0 026 542 B1**

Preparation of esters containing an alpha cyano group in the alcohol portion of the ester

This invention relates to a process for preparing esters of carboxylic acids, more specifically, esters which contain a cyano group bonded to the alpha-carbon atom in the alcohol portion of the ester molecule.

Esters with a cyano group so situated are prepared by reacting an acid with the appropriate cyanohydrin. According to U.S. 3,835,176, the reaction can also be effected by treating an acyl halide with a mixture of the appropriate aldehyde and aqueous sodium or potassium cyanide, optionally in an aprotic solvent. It is disclosed, for example, that 3-phenoxy-$\alpha$-cyanobenzyl chrysanthemate is prepared in 64% yield by reacting chrysanthemoyl chloride, 3-phenoxybenzaldehyde, and an aqueous solution of sodium cyanide at 0°C for 1 hour.

U.S. 4,110,361 discloses a variation of this process which employs, in addition to the acyl halide, the aldehyde, and the water-soluble cyanide, a mixture of water, a water-immiscible aprotic solvent, and a surface active agent as catalyst. Using a preferred non-ionic poly(ethyleneoxy) compound as the catalyst, for example, the commercial insecticidal ester, $\alpha$-cyano-3-phenoxybenzyl 3-(2,2-dichloroethenyl)-2,2-dimethylcyclopropanecarboxylate is prepared in 80% yield at room temperature in a reaction time of two hours.

U.S. 4,110,362 discloses a variation of this process which employs, in addition to the acyl halide, the aldehyde, and the water-soluble cyanide, a mixture of water, a water-immiscible aprotic solvent, and an "onium" e.g., a quaternary ammonium, catalyst. This variation shortens the reaction time and increases the yield sufficiently to make the process a candidate for the commercial production of insecticidal esters. However, the onium catalysts are relatively expensive.

U.S. 4,110,363, discloses a variation of this process which employs, in addition to the acyl halide, the aldehyde, and the water-soluble cyanide, a mixture of water, a water-immiscible aprotic solvent, and a macrocyclic polyether or "crown ether" catalyst. Using the preferred 18-crown-6 as the catalyst, for example, the commercial insecticidal ester, $\alpha$-cyano-3-phenoxybenzyl 3-(2,2-dichloroethenyl)-2,2-dimethylcyclopropanecarboxylate, is prepared in 76% yield at room temperature in a reaction time of two hours.

Were it possible to shorten the reaction time and increase the yield, producing this and other alpha-cyano esters by reacting an acyl halide with an aldehyde and a cyanide would be of great commercial interest. Insecticidal alpha-cyano- esters whose preparations would be facilitated include $\alpha$-cyano - 3 - phenoxybenzyl 3 - (2,2 - dichloroethenyl) - 2,2 - dimethylcyclopropanecarboxylate and $\alpha$ - cyano - 3-phenoxybenzyl 3-(2,2-dibromoethenyl)-2,2-dimethylcyclopropanecarboxylate. Other insecticidal alpha-cano esters of particular interest are $\alpha$-cyano-3-phenoxybenzyl 3-(2-chloro-3,3,3-trifluoropropenyl) 2,2 - dimethylcyclopropanecarboxylate, $\alpha$ - cyano - 3 - phenoxybenzyl 2,2,3,3 - tetramethylcyclopropane-carboxylate, and $\alpha$-cyano-3-phenoxybenzyl 1-(4-chlorophenyl)-3-methylbutanoate.

The invention now relates to such a process for preparing insecticidal $\alpha$-cyano-3-phenoxybenzyl esters by reacting an acyl halide with 3-phenoxybenzaldehyde in a mixture of substantially water-immiscible aprotic solvent and an aqueous solution of water-soluble cyanide salt in the presence of a catalytic amount of rate-promoting agent, which indeed gives the abovementioned $\alpha$-cyano-3-phenoxybenzyl-esters in a short time and in a high yield, by using a catalytic amount of tertiary amines or polyamines, cryptates, amphoteric surfactants selected from aminoalkylsulfonic acids of the structural formula

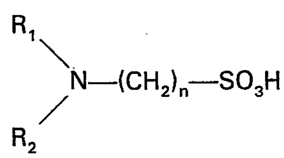

wherein n is 2 or 3, and wherein $R_1$ and $R_2$ are substituents selected collectively to render the aminoalkylsulfonic acids soluble in water-immiscible aprotic solvents and/or acid salts or tertiary amines as the rate promoting agent.

The process of the invention thus is characterized in that an ester is prepared of the formula

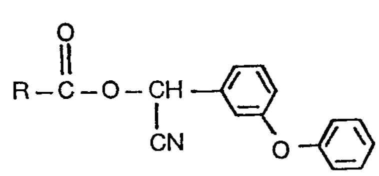

wherein R is selected from 3-(2,2-dichloroethenyl)-2,2-dimethylcyclopropyl, 3-(2,2-dibromoethenyl)-2,2-dimethylcyclopropyl, 3-(2-chloro-3,3,3-trifluoropropenyl)-2,2-dimethylcyclopropyl, 2,2,3,3-tetramethylcyclopropyl, and 1-(4-chlorophenyl)-2-methylpropyl, while using a catalytic amount of tertiary

amines of polyamines, cryptates, amphoteric surfactants selected from aminoalkylsulfonic acids of the structural formula

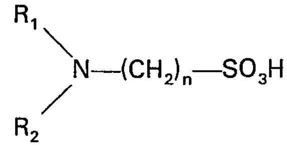

wherein n is 2 or 3, and wherein $R_1$ and $R_2$ are substituents selected collectively to render the aminoalkylsulfonic acids soluble in water-immiscible aprotic solvents and/or acid salts of tertiary amines as the rate promoting agent.

Various aprotic solvents which are substantially water-immiscible may be used in the process. Any alkyl, haloalkyl, aryl, haloaryl, aralkyl, haloaralkyl, or cyclic hydrocarbon, provided that it is a liquid at temperatures between about 0°C and 50°C and forms a discrete second phase when mixed with water, may be used. Such solvents include iso-hexane, 3-methylpentane, 2,3-dimethylbutane, 2,2-dimethylbutane, n-heptane, n-octane, petroleum ether, ligroin, n-propyl bromide, n-propyl iodide, n-butyl chloride, n-butyl bromide, n-pentyl chloride, n-pentyl bromide, diethyl ether, dipropyl ether, dibutyl ether, benzene, toluene, and xylene, for example. Among these solvents, n-heptane is preferred because it is readily available and inexpensive.

A number of water-soluble cyanide salts may be used; for example, the salt may be alkali metal cyanide such as lithium, sodium, potassium, rubidium, or cesium cyanide, or mixtures thereof. Among these, sodium cyanide generally is preferred. However, when it is desired to use certain cryptates as rate-promoting agents, it may be desirable to substitute lithium or potassium cyanide as described below.

The cyanide salt is dissolved in water, the amount of water employed being relatively small, but preferably sufficient to keep all of the cyanide salt in solution under the reaction conditions. In the case that the salt is sodium cyanide, the preferred molar ratio of water to sodium cyanide is between about 3.5 and 6, preferably about 4.5.

The process of this invention is conducted in the presence of a catalytic amount of rate-promoting agent selected from tertiary amines, polyamines, cryptates, amphoteric surfactants as herein before defined and/or acid salts of tertiary amines. For purposes of this invention, a catalytic amount of rate-promoting agent is in the range 1—5 mole percent based on aldehyde, advantageously about 2 mole percent.

The rate-promoting agent may be a tertiary amine or polyamine, a tertiary polyamine being a compound containing more than one tertiary amino nitrogen atom. Particularly desirable tertiary polyamines are linear tertiary polyamines of the formula

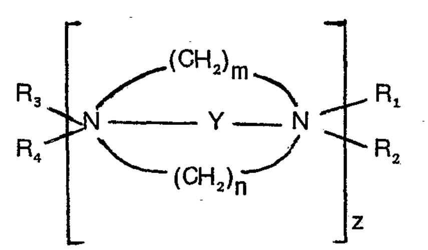

wherein Y is $—(CH_2)_k—$ with k being 1—6, $C_3$—$C_7$ cycloalkane, $C_2$—$C_6$ alkenyl, or $C_2$—$C_6$ alkynyl; z is 1 or 2, and when z is 1, m and n are 0 or independently 1—6, and when m and n are 0, $R_1$, $R_2$, $R_3$ and $R_4$ are hydrocarbon groups, and $R_1$ may be joined with $R_2$, and $R_3$ may be joined with $R_4$ to form a ring containing the N atom to which both are joined, and when m is 1—6 and n is 0, $R_1$ and $R_3$ are absent, and $R_2$ and $R_4$ are hydrocarbon groups, and when both m and n are at least 1, $R_2$ and $R_4$ are absent; and when z is 2, m and n are 0, $R_1$ and $R_3$ are hydrocarbon groups and $R_2$ and $R_4$ are absent.

Particularly useful linear tertiary polyamines within the aforesaid description are 2,4-dimethyl-2,4-diazapentane, 2,5-dimethyl-2,5-diazahexane, 1,1′-(1,2-ethanediyl)bis [piperidine], N,N,N′,N′-tetramethyl-1,2-diaminocyclohexane, 1,4-dimethyl-1,4-diazacyclohexane, diazabicyclo[2,2,2]octane, 2,6-dimethyl - 2,6 - diazaheptane, 2,7 - dimethyl - 2,7 - diazaoctane, 2,7 - dimethyl - 2,7 - diaza - 4 - octene, 2,7-dimethyl-2,7-diaza 4-octyne, 2,9-dimethyl-2,9-diazadecane, and 2,5,8,11-tetramethyl-2,5,8,11-tetraazadodecane. Among these compounds, diazabicyclo-[2,2,2]octane, 2,6-dimethyl-2,6-diazaheptane, 2,7-dimethyl-2,7-diazaoctane, and 2,5,8,11-tetramethyl-2,5,8,11-tetrazadecane are preferred, and diazabicyclo-[2.2.2.]octane is especially attractive.

Macrocyclic tertiary polyamines such as 1,4,8,11-tetraazacyclotetradecane, for example, are also useful, as are sparteine and hexamethylenetetraamine.

Cryptates, polyoxadiazamacrobicycles, constitute another class of rate-promoting agent useful in the practice of this invention. Cryptates of the formula

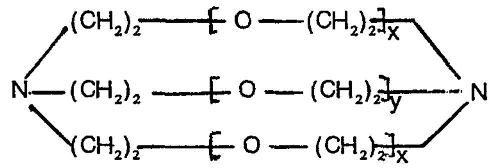

wherein x and y are independently 1 or 2 are especially useful. When the cyanide salt is sodium cyanide 4,7,13,16,21-pentaoxa-1,10-diazabicyclo[8.8.5]tricosane is preferred, but when lithium cyanide is employed 4,7,13,18-tetraoxa-1,10-diazabicyclo[8,5,5]eicosane should be used, and 4,7,13,16,21,24-hexaoxy-1,10-diazabicyclo-[8.8.8]hexacosane is preferred when potassium cyanide is employed.

"Aminoalkylsulfonic acids", having the structural formula

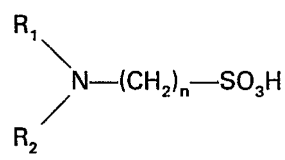

wherein n is 2 or 3, and wherein $R_1$ and $R_2$ are substituents selected collectively to render the aminoalkyl-sulfonic acids soluble in water-immiscible aprotic solvents form a third class of rate-promoting agents. Suitable substituent groups, which collectively contain hydrocarbon units, are present in the following specific aminoalkylsulfonic acids: 1,4-piperazinebis-ethanesulfonic acid, 4-pyridine - ethanesulfonic acid, 2 - [N,N - di - (2 - hydroxy)ethyl]aminoethanesulfonic acid, 3-(cyclohexylamino)propanesulfonic acid, 2-[4-(2-hydroxyethyl)piperizine-1-yl]ethanesulfonic acid, 2-(N-morpholino)ethanesulfonic acid, N-tris(hydroxymethyl)methyl-2-aminoethanesulfonic acid, 2-(N-morpholino)propanesulfonic acid, and N-tris(hydroxymethyl)methyl-2-aminoethanesulfonic acid. Among these compounds, 1,4-piperizinebisethanesulfonic acid and 3-(cyclohexylamino)propanesulfonic acid are preferred, and 3-(cyclohexylamino)-propanesulfonic acid is especially effective.

The tertiary amines whose acid salts are rate-promoting agents contain one or more, e.g., two, nitrogen atoms. For purposes of this invention and wherever it appears in the specification or claims the term, "acid salts of tertiary amines," means products of the reaction between a strong acid, such as HCl, HBr, $H_2SO_4$, $HBF_4$, $HClO_4$, or HCN and a tertiary amine or polyamine, a tertiary polyamine being a compound containing more than one tertiary amino nitrogen atom.

For purposes of this invention, a tertiary amine has the structural formula

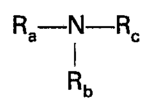

wherein $R_a$, $R_b$, and $R_c$ are hydrocarbon groups.

Particularly desirable tertiary polyamines within the scope of this invention are linear tertiary polyamines of the formula

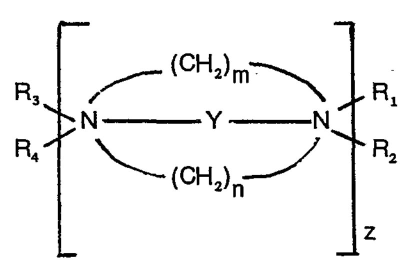

wherein Y is $-(CH_2)_k-$ with k being 1—6, $C_3$—$C_7$ cycloalkane, $C_2$—$C_6$ alkenyl, or $C_2$—$C_6$ alkynyl; z is 1 or 2, and when z is 1, m and n are 0 or independently 1—6, and when m and n are 0, $R_1$, $R_2$, $R_3$, and $R_4$ are hydrocarbon groups, and $R_1$ may be joined with $R_2$, and $R_3$ may be joined with $R_4$ to form a ring containing the N atom to which both are joined, and when m is 1—6 and n is 0, $R_1$ and $R_3$ are absent, and $R_2$ and $R_4$ are hydrocarbon groups, and when both m and n are at least 1, $R_2$ and $R_4$ are absent; and when z is 2, m and n are 0, $R_1$ and $R_3$ are hydrocarbon groups and $R_2$ and $R_4$ are absent.

Particularly useful linear tertiary polyamines within the aforesaid description are 2,4-dimethyl-2,4-diazapentane, 2,5-dimethyl-2,5-diazahexane, 1,1′-(1,2-ethanediyl)bis[piperidine], N,N,N′,N′-tetramethyl-1,2-diaminocyclohexane, 1,4-dimethyl-1,4-diazacyclohexane, diazabicyclo[2.2.2]octane, 2,6-dimethyl-2,6-diazaheptane, 2,7-dimethyl-2,7-diazaoctane, 2,7-dimethyl-2,7-diaza-4-octene, 2,7-dimethyl-2,7-diaza-4-octyne, 2,9-dimethyl-2,9-diazadecane, and 2,5,8,11-tetramethyl-2,5,8,11-tetraazadodecane. Among these compounds, diazabicyclo[2.2.2]octane, 2,6-dimethyl-2,6-diazaheptane, 2,7-dimethyl-2,7-diazaoctane, and 2,5,8,11-tetramethyl-2,5,8,11-tetrazadecane are preferred, and diazabicyclo[2.2.2]octane is especially attractive.

Macrocyclic tertiary polyamines such as 1,4,8,11-tetraazacyclotetradecane, for example, are also useful, as are sparteine and hexamethylenetetraamine.

Although other acid salts are effective, it is preferred that the salt be a hydrohalide, especially a hydrochloride. Acid salts of tertiary amines which may be employed in this invention include, for example, diazabicyclo[2.2.2]octane dihydrochloride, 2,7-dimethyl-2,7-diazaoctane dihydrochloride, 2,3,4,6,7,8,9,10-octahydropyrimido[1,2-*a*]azepine hydrochloride, and quinuclidine hydrochloride.

The process of this invention is carried out between approximately equimolar amounts of the acyl halide, preferably the acyl chloride, aldehyde and aqueous solution of cyanide salt in the water-immiscible aprotic solvent, but slight excesses of the acyl halide and cyanide salt are typically used. The acyl halide may be added last, preferably dropwise, to the stirred reaction mixture, but it is preferred to add a solution containing aldehyde and acyl halide to a stirred mixture of aqueous cyanide salt and water-immiscible aprotic solvent. Although the reaction can be carried out over a wide temperature range, the range 0°C—50°C is satisfactory in most cases, and it is preferred to carry out the reaction at room temperature, since neither external heating nor cooling are then required.

The process will be understood more readily by reference to the following Examples, which illustrate it. Temperatures are in degrees Celsius. The reactions exemplified were, in many cases, monitored by gas liquid partition chromatography (glpc), and the time required for disappearance of the limiting reagent after beginning addition of the acyl halide was determined, together with the amount of alpha-cyano ester produced at that time.

## Example 1

Preparation of $\alpha$ - cyano - 3 - phenoxybenzyl 3 - (2,2 - dichloroethenyl) - 2,2 - dimethylcyclopropanecarboxylate

A. Using diazabicyclo[2.2.2]octane

(1) A flask was charged with 3-phenoxybenzaldehyde (1.98 g, 10.0 mmole), sodium cyanide (0.59 g, 12 mmole), 20 ml n-heptane, 1 ml water, and diazabicyclo[2.2.2]octane (0.022 g, 0.2 mmole). This mixture was vigorously stirred, and 3-(2,2-dichloroethenyl)-2,2-dimethylcyclopropanecarbonyl chloride (2.38 g, 10.5 mmole) was added in one portion. Stirring was continued at room temperature for one hour, at which time glpc indicated a 94% yield of the desired alpha-cyano ester. The mixture was filtered. The filter cake was washed twice with 10 ml portions of diethyl ether. The filtrate was dried over magnesium sulfate, and the heptane was stripped under reduced pressure to yield the desired ester as a residual oil (4.10 g).

Use of the optically active (1R,*cis*) carbonyl chloride in the aforesaid process afforded the corresponding optically active ester in 96% yield within 1 hr. reaction time.

(2) Under a dry nitrogen atmosphere a mixture of sodium cyanide (5.9 g, 0.12 mole) and 1,4-diazabicyclo[2.2.2]octane (0.22 g, 0.002 mole) in 10 grams of water was stirred at room temperature. During a one hour period a solution of 3-phenoxybenzaldehyde (20.6 g, 0.1 mole) and 3-(2,2-dichloroethenyl)-2, 2-dimethylcyclopropanecarbonyl chloride (24.1 g, 0.105 mole) in 120 ml of n-octane was added to the reaction mixture. After complete addition, the reaction mixture was stirred for one hour. An aqueous solution containing 20% sodium carbonate was added to the reaction mixture and the total warmed to 60°. The organic phase was separated and washed with water, dried over anhydrous magnesium sulfate and filtered. The solvent was removed from the filtrate under reduced pressure to yield $\alpha$-cyano-3-phenoxybenzyl 3-(2,2-dichloroethenyl)-2,2-dimethylcyclopropanecarboxylate.

B. Using 2,6-dimethyl-2,6-diazaheptane

A flask was charged with a solution of 3-phenoxybenzaldehyde (1.98 g, 10 mmole) in 10 ml n-heptane. This solution was cooled to 10°, then 2,6-dimethyl-2,6-diazaheptane (26 mg, 0.2 mmole), sodium cyanide (0.59 g, 12 mmole), and 1 ml water were added. 3-(2,2-Dichloroethenyl)-2,2-dimethylcyclopropanecarbonyl chloride (2.38 g, 10.5 mmole) was then added in one portion with stirring, and the temperature of the reaction mixture was maintained at 8—12°. After 45 minutes, glpc indicated at 93% yield of the desired alipha-cyano ester. After a total of 1.25 hours, the reaction mixture was filtered. The filter cake was washed with ether, and the solvent was evaporated from the filtrate, affording the desired ester (4.3 g).

C. Using 2,5,8,11-tetramethyl-2,5,8,11-tetraazadodecane

A flask equipped with a stirrer, addition funnel, and an inlet for nitrogen gas was charged with sodium cyanide (0.59 g, 0.012 mole) dissolved in 1 ml water, 2,5,8,11-tetramethyl-2,5,8,11-tetraazadodecane (0.048 g, 0.2 mmole) in 3 ml heptane, and 3-phenoxybenzaldehyde (1.98 g, 0.01 mole) in 7 ml heptane. The reactants were mixed under a nitrogen atmosphere. 3-(2,2-Dichloroethenyl)-2,2-dimethylcyclopropanecarbonyl chloride (2.38 g, 0.0105 mole) in 10 ml heptane was added dropwise to the stirred mixture over a period of 20 minutes. Twenty minutes later, a total of 40 minutes, glpc

indicated a 93% yield of the desired ester. The reaction mixture was stirred for another 40 minutes, then poured into a separatory funnel, diluted with 40 ml diethyl ether, washed once with a 1N aqueous solution of sodium hydroxide, three times with water, and once with saturated aqueous sodium chloride solution. After phase separation, the separated ethereal layer was dried over magnesium sulfate, and the solvent was evaporated, affording the ester as a pale yellow oil (4.02 g).

D. Using 4,7,13,16,21-pentaoxa-1,10-diazabicyclo[8.8.5]tricosane

A flask was charged with 3-phenoxybenzaldehyde (1.98 g, 10 mmole), 10 ml n-heptane, 4,7,13,16,21-pentaoxa-1,10-diazabicyclo[8.8.5]tricosane (66 mg, 0.2 mmole), sodium cyanide (0.59 g, 12 mmole), 1 ml water, and a solution of 3-(2,2-dichloroethenyl)-2,2-dimethylcyclopropanecarbonyl chloride (2.38 g, 10.5 mmole) in 10 ml n-heptane. The reaction mixture was stirred, and an exotherm (to approximately 40°) was observed after 20 minutes. After 50 minutes, glpc showed a 99% yield of the desired ester. The reaction mixture was filtered, diluted with ether, dried over magnesium sulfate, and the solvent was evaporated to afford the desired ester (3.90 g).

E. Using Tetramethylethylene Diamine

A solution of 3-(2,2-dichloroethenyl)-2,2-dimethylcyclopropanecarbonyl chloride (114 g, 0.315 mole) and 3-phenoxybenzaldehyde (61.9 g, 0.3 mole) in 275 ml of n-heptane was added dropwise under nitrogen in one hour to a stirred mixture of sodium cyanide (18 g, 0.36 mole), tetramethylethylene diamine (0.7 g, 0.006 mole), and 30 g of water at about 40°. After the addition, the reaction mixture was stirred for 90 minutes at about 40°. The reaction mixture was worked up as in Example 1A.(2), producing the desired ester.

F. Using Tetramethyl-1,6-hexane Diamine

By the method of Example 1E. above, but substituting tetramethyl-1,6-hexane diamine for tetramethylethylene diamine, $\alpha$ - cyano - 3 - phenoxybenzyl 3 - (2,2 - dichloroethenyl) - 2,2 - dimethylcyclopropanecarboxylate was prepared.

The preparation of $\alpha$ - cyano - 3 - phenoxybenzyl 3 - (2,2 - dichloroethenyl) - 2,2 - dimethylcyclopropanecarboxylate using other tertiary amine or polyamine rate-promoting agents under otherwise similar conditions gave the results shown in Table 1.

Example 2

Preparation of $\alpha$-cyano-3-phenoxybenzyl 3-(2,2-dibromoethenyl)-2,2-dimethylcyclopropanecarboxylate using diazabicyclo[2.2.2]octane

A flask was charged with 3-phenoxybenzaldehyde (1.98 g, 10.0 mmole), 10 ml n-heptane, diazabicyclo[2.2.2]octane (22 ml, 0.2 mmole), sodium cyanide (0.59 g, 12 mmole), and 1 ml water. 3-(2,2-Dibromoethenyl)-2,2-dimethylcyclopropanecarbonyl chloride (3.32 g, 10.5 mmole) in 10 ml n-heptane was added with stirring. After 2 hours, the reaction mixture was filtered, the filter cake was washed with 30 ml of ether, and the filtrate was dried over magnesium sulfate. The solvent was stripped, affording the desired ester as a yellow oil (4.4 g, 96% yield).

Example 3

Preparation of $\alpha$-cyano-3-phenoxybenzyl 3-(2-chloro-3,3,3-trifluoropropenyl)-2,2-dimethylcyclopropanecarboxylate using 2,7-dimethyl-2,7-diazaoctane

A flask was charged with 3-phenoxybenzaldehyde (1.98 g, 10 mmole), 10 ml n-heptane, 2,7-dimethyl-2,7-diazaoctane (29 mg, 0.2 mmole), sodium cyanide (0.59 g, 12 mmole) and 1 ml water. 3-(2-Chloro-3,3,3-trifluoropropenyl)-2,2-dimethylcyclopropanecarbonyl chloride (2.0 g, 10.5 mmole) dissolved in 10 ml n-heptane was then added in one portion. After 1.3 hr., the conversion was complete. The reaction mixture was stirred for 4.5 hr, when 5 ml diethyl ether and 5 ml 1N aqueous sodium hydroxide solution were added. The resultant mixture was stirred for 1.5 hr and the two phases separated. The organic phase was washed once with 1N aqueous sodium hydroxide solution, once with water, and once with a saturated aqueous sodium chloride solution, then dried over magnesium sulfate, filtered, and the solvent distilled off under reduced pressure to afford the desired ester (4.1 g, 91% yield).

Example 4

Preparation of $\alpha$-cyano-3-phenoxybenzyl 2,2,3,3-tetramethylcyclopropanecarboxylate using 2,6-dimethyl-2,6-diazaheptane

A flask was charged with 3-phenoxybenzaldehyde (3.16 g, 16 mmole), 10 ml n-heptane, 2,6-dimethyl-2,6-diazaheptane (26 mg, 0.2 mmole), sodium cyanide (0.94 g, 19.2 mmole) and 1 ml water. 2,2,3,3-Tetramethylcyclopropanecarbonyl chloride (2.6 g, 16.7 mmole) dissolved in 10 ml n-heptane was added next, in one portion. An exotherm (to approximately 38°) was observed upon addition of the acid chloride. Analysis indicated a 74% yield of the desired ester in 4 hours. After cooling, the reaction mixture was filtered, diluted with diethyl ether, dried over magnesium sulfate, and distilled under reduced pressure to afford an orange residual oil. The oil was dissolved in 25 ml diethyl ether, and the

resultant ethereal solution was mixed for 2 hr with a 1N aqueous sodium hydroxide solution. After phase separation, the ethereal layer was washed once with water, once with a saturated aqueous sodium chloride solution, dried over magnesium sulfate, and the solvent was distilled under reduced pressure to afford the desired ester (3.0 g).

Example 5

Preparation of α-cyano-3-phenoxybenzyl 2-(4-chlorophenyl)-3-methyl butanoate

A. Using diazabicyclo[2.2.2]octane

A flask was charged with 3-phenoxybenzaldehyde (1.98 g, 10 mmole), 10 ml n-heptane, diazabicyclo[2.2.2]octane (22 mg, 0.2 mmole), sodium cyanide (0.59 g, 12 mmole), and 1 ml water, and stirring was begun. 2-(4-Chlorophenyl)-3-methylbutanoyl chloride (2.42 g, 10.5 mmole) in 10 ml n-heptane was then added in one portion. The reaction mixture was stirred at room temperature for 1 hr at which time glpc indicated at 74% yield of the desired ester. Stirring was continued overnight, then the reaction mixture was filtered, diluted with diethyl ether, and the solvent was distilled under reduced pressure to afford the desired ester as the residue (4.02 g).

B. Using 2,5,8,11-tetramethyl-2,5,8,11-tetraazadodecane

A flask was charged with 3-phenoxybenzaldehyde (1.98 g, 10 mmole), 20 ml n-heptane, 2,5,8,11-tetramethyl-2,5,8,11-tetraazadodecane (0.046 g), sodium cyanide (0.59 g, 12 mmole), and 1 ml. water. With stirring, 2-(4-chlorophenyl)-3-methylbutanoyl chloride (2.42 g, 10.5 mmole) was added dropwise over a period of 6 minutes. The reaction mixture was stirred at room temperature. Analysis by glpc 2.4 hr. after the acyl chloride has been added indicated a 67.7% yield of the desired ester. Stirring was continued overnight, and the desired ester (3.4 g) was isolated as described in the preceding Example

Example 6

Preparation of α-cyano-3-phenoxybenzyl 3-(2,2-dichloroethenyl)-2,2-dimethylcyclopropanecarboxylate

A. Using 1,4-piperazinebisethanesulfonic acid as the rate-promoting agent

A flask was charged with 3-phenoxybenzaldehyde (1.98 g, 10 mmole), 10 ml n-heptane, 1,4-piperazinebisethanesulfonic acid (60 mg, 0.2 mmole), sodium cyanide (0.59 g, 12 mmole), 1 ml water, and a solution of 3-(2,2-dichloroethenyl)-2,2-dimethylcyclopropanecarbonyl chloride (2.38 g, 10.5 mmole) in 10 ml n-heptane. The reaction mixture was stirred for 4.5 hours (glpc indicated at 91% yield after 1 hr, 50 min), diluted with 30 ml diethyl ether, then washed once with 1N aqueous sodium hydroxide solution (20 ml), once with water (20 ml), and once with a saturated aqueous sodium chloride solution (20 ml). After separation, the clear yellow ethereal solution was dried over magnesium sulfate; then the solvent was evaporated to afford the desired ester (4.05 g).

B. using 3-(cyclohexylamino)propanesulfonic acid as the rate-promoting agent

(1) In the manner of Example A above, but substituting 3-(cyclohexylamino)propanesulfonic acid (44 mg, 0.2 mmole) for the 1,4-piperazinebisethanesulfonic acid, glpc indicated a 97.2% yield of the desired ester after 95 minutes and isolation afforded the desired ester (3.49 g).

In a similar experiment, glpc indicated a 99% yield of the desired ester in two hours.

(2) A stirred mixture of sodium cyanide (18.0 g, 0.36 mole) and 3-(cyclohexylamino)propanesulfonic acid (1.34 g, 0.006 mole) in 300 ml of water was warmed to 40°. During a one hour period, maintaining a reaction temperature of about 40°, a solution of *cis*-3-(2,2-dichloroethenyl)-2,2-dimethylcyclopropanecarbonyl chloride (71.7 g, 0.315 mole) and 3-phenoxybenzaldehyde (61.9 g, 0.3 mole) in 262.2 g of n-heptane was added. The mixture was then stirred at 40°C for one additional hour, after which it was washed with an aqueous solution containing 20% sodium carbonate and then with water. The organic phase was separated from the mixture and the solvent removed by distillation under reduced pressure to give α-cyano-3-phenoxybenzyl *cis*-3-(2,2-dichloroethenyl)-2,2-dimethylcyclopropanecarboxylate.

The substitution of other rate-promoting agents under otherwise similar conditions gave the following yields (glpc) after the indicated reaction times.

| Rate-Promoting Agent | Reaction Time | Yield |
|---|---|---|
| 2-[4-(2-hydroxyethyl)piperazine-1-yl]ethanesulfonic acid | 55 min. | 91% |
| 4-pyridineethanesulfonic acid | 2.3 hr. | 94% |
| 2-[N,N-di-(2-hydroxy)ethyl] aminoethanesulfonic acid | 3.3 hr. | 90% |

Example 7

Preparation of α-cyano-3-phenoxybenzyl 2-(4-chlorophenyl)-3-methyl butanoate using 3-(cyclohexylamino)propanesulfonic acid as the rate-promoting agent

A flask was charged with 3-(cyclohexylamino)propanesulfonic acid (42 mg), 3-phenoxybenzaldehyde (1.90 g, 9.6 mmole), sodium cyanide (0.56 g, 12 mmole), 1 ml water, and 15 ml n-heptane. 2-(4-Chlorophenyl)-3-methylbutanoyl chloride (2.34 g, 10.1 mmole) in 5 ml n-heptane was added dropwise over a period of 24 minutes to the stirred mixture. Thirty minutes after the addition was complete, a total of 54 minutes, glpc indicated a 95.6% yield of the desired ester. After stirring overnight, the reaction mixture was filtered and extracted with ether. The ether was evaporated from the extract, affording α-cyano-3-phenoxybenzyl 2-(4-chlorophenyl)-3-methyl butanoate (3.47 g).

Example 8

Preparation of α-cyano-3-phenoxybenzyl 3-(2,2-dichloroethenyl) 2,2-dimethylcyclopropanecarboxylate using diazabicyclo[2.2.2]octane dihydrochloride as the rate-promoting agent

(1) A flask was charged with 3-phenoxybenzaldehyde (1.98 g, 10.0 mmole) 10 ml n-heptane, diazabicyclo[2.2.2]octane dihydrochloride (30 mg, 0.2 mmole), sodium cyanide (0.59 g, 12 mmole), 1 ml water, and a solution of 3-(2,2-dichloroetheny)-2,2-dimethylcyclopropanecarbonyl chloride (2.38 g, 10.5 mmole) in 10 ml n-heptane. The reaction mixture was stirred, and after 1.5 hours glpc indicated a 96% yield of the desired ester. After a total of 1 hr., 50 min, the reaction mixture was filtered, diluted with ether, the phases were separated, the ether phase was dried over magnesium sulfate, and the solvent was evaporated to afford the desired ester (3.98 g).

(2) A stirred mixture of sodium cyanide (18.1 g, 0.36 mole) and diazabicyclo[2.2.2]octane dihydrochloride (1.11 g, 0.006 mole) in 30 g of water was warmed to 40°. During a one hour period a solution of 3-(2,2-dichloroethenyl)-2,2-dimethylcyclopropanecarbonyl chloride (77.6 g, 0.33 mole) and 3-phenoxybenzaldehyde (61.5 g, 0.3 mole) in 102 ml of n-heptane was added to the reaction mixture. After complete addition, the reaction mixture was stirred for 40 minutes and an additional 3.5 g (0.015 mole) of the acyl chloride added. The reaction mixture was stirred for an additional 40 minutes and then washed with 100 g of a 10% aqueous sodium carbonate solution and then water. The organic phase was separated from the mixture and the solvent removed by distillation under reduced pressure to give α-cyano-3-phenoxybenzyl 3-(2,2-dichloroethenyl)-2,2-dimethylcyclopropanecarboxylate.

The preparation of α-cyano-3-phenoxybenzyl 3-(2,2-dichloroethenyl)-2,2-dimethylcyclopropanecarboxylate using other rate-promoting agents under otherwise similar conditions gave the following results.

| Rate-Promoting Agent | Reaction Time | Yield |
|---|---|---|
| 2,7-dimethyl-2,7-diazaoctane dihydrochloride | 1.4 hr | 96% |
| 2,3,4,6,7,8,10-octahydro-pyrimido[1,2*a*]azepine hydrochloride | 4 hr | 99% |
| quinuclidine hydrochloride | 1.8 hr | 98% |

TABLE 1

Preparation of α-cyano-3-phenoxybenzyl 3-(2,2-dichloroethenyl)-2,2-dimethylcyclopropanecarboxylate using other tertiary amine or polyamine rate-promoting agents

| Rate-Promoting Agent | Reaction Time | Yield |
|---|---|---|
| tri-n-hexylamine | 4.5 hours | 82% |
| triethylamine | 2 hours | 89% |
| 2,4-dimethyl-2,4-diazapentane | 24 hours | 96% |
| 2,5-dimethyl-2,5-diazahexane | 0.5 hour | 91% |
| 2,9-dimethyl-2,9-diazadecane | 1.0 hour | 96% |
| 1,1′-(1,2-ethanediyl)bis[piperidine] | 1.0 hour | 94% |
| 1,4-dimethyl-1,4-diazacyclohexane | 1.0 hour | 86% |
| N,N,N′,N′-tetramethyl-1,2-diaminocyclohexane | 1.5 hours | 96% |
| 1,4,8,11-tetramethyl-1,4,8,11-tetraazacyclotetradecane | 1.5 hours | 96% |
| (—)-sparteine | 1.3 hours | 95% |

## Claims

1. A process for preparing insecticidal α-cyano-3-phenoxybenzyl esters by reacting an acyl halide with 3-phenoxybenzaldehyde in a mixture of substantially water-immiscible aprotic solvent and an aqueous solution of water-soluble cyanide salt in the presence of a catalytic amount of rate-promoting agent characterized in that an ester is prepared of the formula

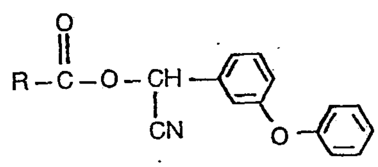

wherein R is selected from 3-(2,2-dichloroethenyl)-2,2-dimethylcyclopropyl, 3-(2,2-dibromoethenyl)-2,2-dimethylcyclopropyl, 3-(2-chloro-3,3,3,-trifluoropropenyl)-2,2-dimethylcyclopropyl, 2,2,3,3,-tetramethylcyclopropyl, and 1-(4-chlorophenyl)-2-methylpropyl, while using a catalytic amount of tertiary amines or polyamines, cryptates, amphoteric surfactants selected from aminoalkylsulfonic acids of the structural formula

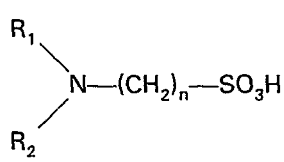

wherein n is 2 or 3, and wherein $R_1$ and $R_2$ are substituents selected collectively to render the aminoalkyl-sulfonic acids soluble in water-immiscible aprotic solvents and/or acid salts of tertiary amines as the rate promoting agent.

2. A process according to claim 1 characterized by using a rate-promoting agent selected from tertiary polyamines which are linear tertiary polyamines of the formula

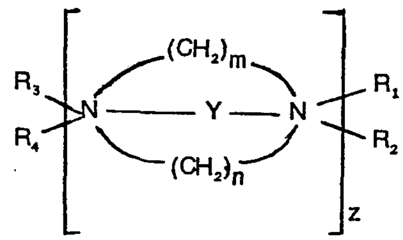

wherein Y is —$(CH_2)_k$— with k being 1—6, $C_3$—$C_7$ cycloalkane, $C_2$—$C_6$ alkenyl, or $C_2$—$C_6$ alkynyl; z is 1 or 2, and when z is 1, m and n are 0 or independently 1—6, and when m and n are 0, $R_1$, $R_2$, $R_3$, and $R_4$ are hydrocarbon groups, and $R_1$ may be joined with $R_2$, and $R_3$ may be joined with $R_4$ to form a ring containing the N atom to which both are joined, and when m is 1—6 and n is 0, $R_1$ and $R_3$ are absent, and $R_2$ and $R_4$ are hydrocarbon groups, and when both m and n are at least 1, $R_2$ and $R_4$ are absent; and when z is 2, m and n are 0, $R_1$ and $R_3$ are hydrocarbon groups and $R_2$ and $R_4$ are absent, 1,4,8,11-tetramethyl-1,4,8,11-tetraazacyclotetradecane, sparteine, and cryptates.

3. The process of claim 2 characterized in that the linear tertiary polyamine is selected from 2,4-dimethyl - 2,4 - diazapentane, 2,5 - dimethyl - 2,5 - diazahexane, 1,1′ - (1,2 - ethanediyl)bis[piperidine], N,N,N′,N′ - tetramethyl - 1,2 - diaminocyclohexane, 1,4 - dimethyl - 1,4 - diazacyclohexane, diazabicyclo[2.2.2]octane, 2,6-dimethyl-2,6-diazaheptane, 2,7-dimethyl-2,7-diazaoctane, 2,9-dimethyl-2,9-diazadecane, and 2,5,8,11-tetramethyl-2,5,8,11-tetraazadodecane.

4. The process of claim 3 characterized in that the linear tertiary polyamine is diazabicyclo[2.2.2]-octane, 2,6-dimethyl-2,6-diazaheptane, 2,7-dimethyl-2,7-diazaoctane, or 2,5,8,11-tetramethyl-2,5,8,11-tetraazadecane.

5. The process of claim 4 characterized in that the rate promoting agent is diazabicyclo[2.2.2]octane.

6. The process of claim 2 characterized in that the rate promoting agent is a cryptate of the formula

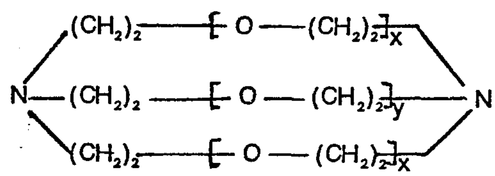

wherein x and y are independently 1 or 2.

7. The process of claim 6 characterized in that the cryptate is 4,7,13,16,21,-pentaoxo-1,10-diazabicyclo-[8.8.5]tricosane.

8. A process according to any one of claims 2—7 characterized in that R is 3-(2,2-dichloroethenyl)-2,2-dimethylcyclopropyl.

9. A process according to any one of claims 2—7 characterized in that the water-immiscible aprotic solvent is n-heptane.

10. A process according to any one of claims 2—7 characterized in that the water-soluble cyanide salt is sodium cyanide.

11. The process of claim 1 characterized in that the rate-promoting agent is an aminoalkylsulfonic acid selected from 1,4-piperazinebisethanesulfonic acid, 4-pyridineethanesulfonic acid, 2-[N,N-di-(2-hydroxy)-ethyl] aminoethanesulfonic acid, 3-(cyclohexylamino)propanesulfonic acid, and 2-[4-(2-hydroxyethyl)piperizine-1-yl]ethanesulfonic acid.

12. The process of claim 11 characterized in that the rate-promoting agent is selected from 1,4-piperizine-bisethanesulfonic acid and 3-(cyclohexylamino)propane-sulfonic acid.

13. The process of claim 12 characterized in that the rate-promoting agent is 3-(cyclohexylamino)propane-sulfonic acid.

14. A process according to any one of claims 11—13 characterized in that R is 3-(2,2-dichloroethenyl)-2,2-dimethylcyclopropyl or 1-(4-chlorophenyl)-2-methylpropyl.

15. A process according to any one of claims 11—13 characterized in that R is 3-(2,2-dichloroethenyl)-2,2-dimethylcyclopropyl.

16. A process according to any one of claims 11—13 characterized in that the water-immiscible aprotic solvent is n-heptane.

17. A process according to any one of claims 11—13 characterized in that the water-soluble cyanide salt is sodium cyanide.

18. The process of claim 1 characterized in that the rate-promoting agent is an acid salt of a tertiary amine selected from products of the reaction of a strong acid and a linear tertiary polyamine of the formula

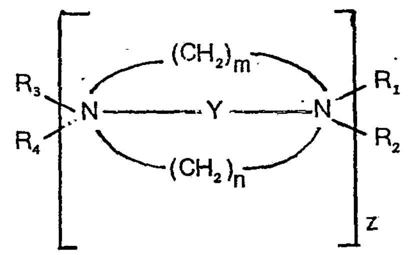

wherein Y is $-(CH_2)_k-$ with k being 1—6, $C_3$—$C_7$ cyclo-alkane, $C_2$—$C_6$ alkenyl, or $C_2$—$C_6$ alkynyl; z is 1 or 2, and when z is 1, m and n are 0 or independently 1—6, and when m and n are 0, $R_1$, $R_2$, $R_3$, and $R_4$ are hydrocarbon groups, and $R_1$ may be joined with $R_2$, and $R_3$ may be joined with $R_4$ to form a ring containing the N atom to which both are joined, and when m is 1—6 and n is 0, $R_1$ and $R_3$ are absent, and $R_2$ and $R_4$ are hydrocarbon groups, and when both m and n are at least 1, $R_2$ and $R_4$ are absent; and when z is 2, m and n are 0, $R_1$ and $R_3$ are hydrocarbon groups and $R_2$ and $R_4$ are absent.

19. The process of claim 18 characterized in that the rate-promoting agent is selected from diazabicyclo[2.2.2]octane dihydrochloride, 2,7 - dimethyl - 2,7 - diazaoctane dihydrochloride, 2,3,4,6,7,8,9,10 - octahydropropyrimido[1,2-*a*]azepine hydrochloride, and quinuclidine hydrochloride.

20. A process of claim 19 characterized in that the rate-promoting agent is diazabicyclo[2.2.2]octane dihydrochloride.

21. A process according to any one of claims 18—20 characterized in that R is 3-(2,2-dichloroethenyl)-2,2-dimethylcyclopropyl.

22. A process according to any one of claims 18—20 characterized in that the water-immiscible aprotic solvent is n-heptane.

23. A process according to any one of claims 18—20 characterized in that the water-soluble cyanide salt is sodium cyanide.

## Patentansprüche

1. Verfahren zur Herstellung insektizider $\alpha$-Cyano-3-phenoxybenzylester durch die Reaktion eines Acylhalids mit 3-Phenoxybenzaldehyd in einem Gemisch eines überwiegend wasserunmischbaren aprotischen Lösungsmittels und einer wässrigen Lösung eines wasserlöslichen Cyanidsalzes in Gegenwart einer katalytischen Menge eines die Geschwindigkeit förderndes Mittels, dadurch gekennzeichnet, dass ein Ester der Formel

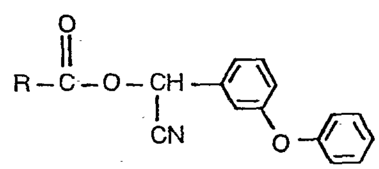

hergestellt wird, in der R aus 3-(2,2-Dichloroäthenyl)-2,2-dimethylcyclopropyl, 3-(2,2-Dibromoäthenyl) - 2,2 - dimethylcyclopropyl, 3 - (2 - Chloro - 3,3,3 - trifluoropropenyl) - 2,2 - dimethylcyclopropyl, 2,2,3,3-Tetramethylcyclopropyl, und 1-(4-Chlorophenyl)-2-methylpropyl gewählt wird, unter Anwendung einer katalytischen Menge tertiärer Amine oder Polyamide, Kryptate, amphoterische oberflächen aktive Mittel, gewählt aus Aminoalkylsulfon Säuren der Strukturformel

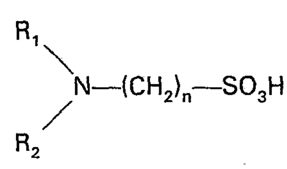

in der n 2 oder 3 ist und in der $R_1$ und $R_2$ Substituenten sind die sämtlich so gewählt worden sind dass die Aminoalkylsulfon Säuren löslich sind in wasserunmischbaren aprotischen Lösungsmitteln und/oder säuren Salzen der tertiären Aminen als Geschwindigkeit förderndes Mittel.

2. Verfahren nach Anspruch 1, dadurch gekennzeichnet, dass ein Geschwindigkeit förderndes Mittel verwendet wird dass gewählt worden ist aus den linären tertiären Polyaminen der Formel

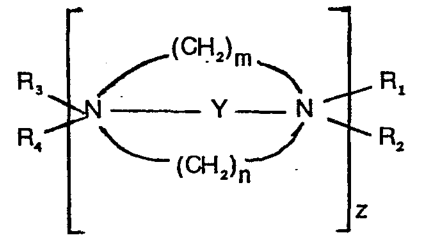

in der Y $-(CH_2)_k-$ mit k 1—6, $C_3$—$C_7$-Cycloalkan, $C_2$—$C_6$-Alkenyl oder $C_2$—$C_6$ Alkynyl ist, Z ist 1 oder 2, und falls z 1 ist, m und n 0 oder unabhängig 1—6 sind, und falls m und n 0 sind, $R_1$, $R_2$, $R_3$ Kohlenwasserstoffgruppen sind, wobei $R_1$ mit $R_2$ verbunden sein kann, und $R_3$ mit $R_4$ verbunden sein kann unter Bildung eines Ringes, der das N-Atom enthält, mit welchem beide verbunden sind, und falls m 1—6 ist und n 0 ist, $R_1$ und $R_3$ abwesend sind, und $R_2$ und $R_4$ Kohlenwasserstoffgruppen sind, und falls m und n beide mindestens 1 sind, $R_2$ und $R_4$ abwesend sind; und falls z 2 ist, m und n 0 sind, $R_1$ und $R_3$ Kohlenwasserstoffgruppen sind und $R_2$ und $R_4$ abwesend sind, aus den 1,4,8,11-Tetramethyl-1,4,8,11-tetraazacyclotetradecane, Sparteine und Kryptate.

3. Verfahren nach Anspruch 2, dadurch gekennzeichnet, dass das lineäre tertiäre Polyamin aus 2,4 - Dimethyl - 2,4 - diazapentan, 2,5 - Dimethyl - 2,5 - diazahexan, 1,1' - (1,2 - Äthanediyl) - bis-[piperidin], N,N,N',N'-Tetramethyl-1,2-diaminocyclohexan, 1,4-Dimethyl-1,4-diazacyclohexan, Diazabicyclo[2,2,2]-octan, 2,6-Dimethyl-2,6-diazaheptan, 2,7-Dimethyl-2,7-diazaoctan, 2,9-Dimethyl-2,9-diazadecan, und 2,5,8,11-Tetramethyl-2,5,8,11-Tetraazadodecan gewählt ist.

4. Verfahren nach Anspruch 3, dadurch gekennzeichnet, dass das lineäre tertiäre Polyamin Diazabicyclo[2,2,2]-octan, 2,6-Dimethyl-2,6-diazaheptan, 2,7-Dimethyl-2,7-Diazaoctan oder 2,5,8,11-Tetramethyl-2,5,8,11-Tetraazadecan ist.

5. Verfahren nach Anspruch 4, dadurch gekennzeichnet, dass das die Geschwindigkeit förderndes Mittel Diazabicyclo[2,2,2]octan ist.

6. Verfahren nach Anspruch 2, dadurch gekennzeichnet, dass das die Geschwindigkeit förderndes Mittel ein Kryptat der Formel

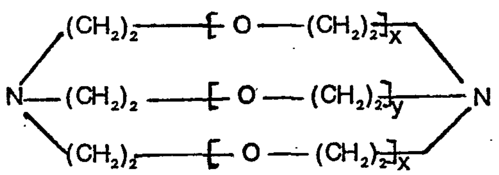

ist, in der x und y unabhängig 1 oder 2 sind.

7. Verfahren nach Anspruch 6, dadurch gekennzeichnet, dass das Kryptat 4,7,13,16,21-pentaoxo-1,10-Diazabicyclo-[8,8,5] tricosan ist.

8. Verfahren nach einem der Ansprüche 2—7, dadurch gekennzeichnet, dass R 3-(2,2-Dichloroäthenyl)-2,2-dimethylcyclopropyl ist.

9. Verfahren nach einem der Ansprüche 2—7, dadurch gekennzeichnet, dass das wasserunmischbare aprotische Lösungsmittel n-Heptan ist.

10. Verfahren nach einem der Ansprüche 2—7, dadurch gekennzeichnet, dass das wasserlösliche Cyanidsalz Natriumcyanid ist.

11. Verfahren nach Anspruch 1, dadurch gekennzeichnet, dass das die Geschwindigkeit förderndes Mittel eine Aminoalkylsulfonsäure ist, die aus 1,4-Piperazin-bisäthansulfonsäure, 4-Pyridinäthansulfonsäure, 2 - [N,N - di - (2 - hydroxy) - äthyl] - aminoäthansulfonsäure, 3 - (Cyclohexylamino)-propansulfonsäure, und 2-[4-(2-hydroxyäthyl)-piperizin-1-yl]-äthansulfonsäure gewählt ist.

12. Verfahren nach Anspruch 1, dadurch gekennzeichnet, dass das die Geschwindigkeit förderndes Mittel aus 1,4-Piperizin-bisäthansulfonsäure, und 3-(Cyclohexylamino)propansulfonsäure gewählt ist.

13. Verfahren nach Anspruch 12, dadurch gekennzeichnet, dass das die Geschwindigkeit forderndes Mittel 3-(Cyclohexylamino)propansulfonsäure ist.

14. Verfahren nach einem der Ansprüche 11—13, dadurch gekennzeichnet, dass R 3-(2,2-Dichloroäthenyl)-2,2-dimethylcyclopropyl oder 1-(4-Chlorophenyl)-2-methylpropyl ist.

15. Verfahren nach einem der Ansprüche 11—13, dadurch gekennzeichnet, dass R 3-(2,2-Dichloroäthenyl)-2,2-dimethylcyclopropyl ist.

16. Verfahren nach einem der Ansprüche 11—13, dadurch gekennzeichnet, dass das wasserunmischbare aprotische Lösungsmittel N-Heptan ist.

17. Verfahren nach einem der Ansprüche 11—13, dadurch gekennzeichnet, dass das wasserlösliche Cyanidsalz Natriumcyanid ist.

18. Verfahren nach Ansprüche 1, dadurch gekennzeichnet, dass das die Geschwindigkeit förderndes Mittel ein säures Salz eines tertiären Amins ist, gewählt aus Reaktionsprodukten einer

starken Säure und eines linearen tertiären Polyamins der Formel

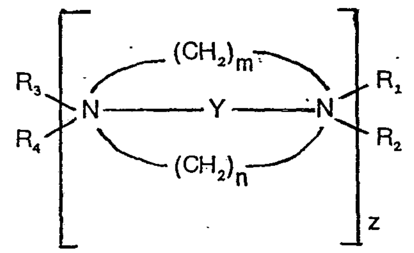

in der Y —$(CH_2)_k$— mit k 1—6, $C_3$—$C_7$ Cycloalkan, $C_2$—$C_6$ Alkenyl, oder $C_2$—$C_6$ Alkynyl ist; z 1 oder 2 ist, und falls z 1 ist, m und n 0 sind oder unabhängig 1—6, und falls m und n 0 sind, $R_1$, $R_2$, $R_3$ und $R_4$ Kohlenwasserstoffgruppen sind, und $R_1$ mit $R_2$ verbunden sein kann, und $R_3$ mit $R_4$ verbunden sein kann unter Bildung eines Ringes, der das N-Atom mit welchem die beiden Verbunden sind, enthält, und falls m 1—6 ist und n 0 ist, $R_1$ und $R_3$ abwesend sind, und $R_2$ und $R_4$ Kohlenwasserstoffgruppen sind, und falls m und n beide mindestens 1 sind, $R_2$ und $R_4$ abwesend sind; und falls z 2 ist, m und n 0 sind, $R_1$ und $R_3$ Kohlenwasserstoffgruppen sind und $R_2$ und $R_4$ abwesend sind.

19. Verfahren nach Anspruch 18, dadurch gekennzeichnet, dass das die Geschwindigkeit förderndes Mittel aus Diazabicyclo[2,2,2]octandihydrochlorid, 2,7-Dimethyl-2,7-diazaoctandihydrochlorid, 2,3,4,6,7,8,9,10-Octahydropropyrimido[1,2-a]azepinhydrochlorid und Chinuclidinhydrochlorid gewählt ist.

20. Verfahren nach Anspruch 19, dadurch gekennzeichnet, dass das die Geschwindigkeit förderndes Mittel Diazabicyclo[2,2,2]octandihydrochlorid ist.

21. Verfahren nach einem der Ansprüche 18—20, dadurch gekennzeichnet, dass R 3-(2,2-Dichloroäthenyl)-2,2-dimethylcyclopropyl ist.

22. Verfahren nach einem der Ansprüche 18—20, dadurch gekennzeichnet, dass das wasserunmischbare aprotische Lösungsmittel n-Heptan ist.

23. Verfahren nach einem der Ansprüche 18—20, dadurch gekennzeichnet, dass das wasserlösliche Cyanidsalz Natriumcyanid ist.

**Revendications**

1. Procédé de préparation d'esters α-cyano-3-phénoxybenzylique insecticides en faisant réagir un halogénure d'acyle avec du 3-phénoxybenzaldéhyde dans un mélange d'un solvant aprotique pratiquement non miscible à l'eau et d'une solution aqueuse d'un cyanure soluble dans l'eau en présence d'une quantité catalytique d'un agent améliorant la vitesse, caractérisé en ce qu'on prépare un ester répondant à la formule:

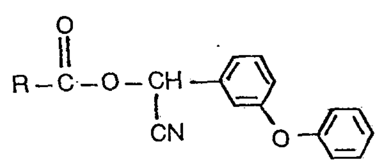

dans laquelle R est choisi parmi les radicaux 3-(2,2-dichloroéthènyl)-2,2-diméthylcyclopropyle, 3-(2,2-dibromoéthènyl)-2,2-diméthylcyclopropyle, 3-(2-chloro-3,3,3-trifluoropropènyl)-2,2-diméthylcyclopropyle, 2,2,3,3,-tétraméthylcyclopropyle, et 1-(4-chlorophényl)-2-méthylpropyle, tout en utilisant une quantité catalytique d'amines ou de polyamines tertiaires, de cryptates, d'agents tensio-actifs amphotères choisis parmi les acides amino-alkyle sulfoniques répondant à la formule développée:

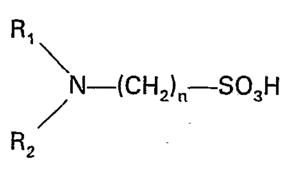

dans laquelle n est égal à 2 ou 3 et $R_1$ et $R_2$ sont des substituants choisis collectivement pour rendre les acides aminoalkyl-sulfonique solubles dans des solvants aprotiques non miscibles à l'eau et/ou des sels d'acids d'amines tertiaires comme agents améliorant la vitesse.

2. Procédé suivant la revendication 1, caractérisé en ce qu'on utilise un agent améliorant la vitesse choisi parmi les polyamines tertiaires qui sont des polyamines tertiaires linéaires répondant à la formule:

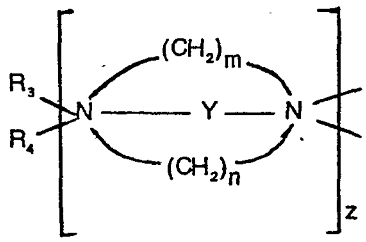

dans laquelle est $-(CH_2)_k-$, k étant un nombre de 1 à 6, un radical cycloalkane en $C_3$ à $C_7$, alcényle en $C_2$ à $C_6$, ou alcynyle en $C_2$ à $C_6$; z est égal à 1 ou 2, et lorsque z est égal à 1, m et n sont égaux à 0 ou sont indépendamment des nombres de 1 à 6, et lorsque m et n sont égaux à 0, $R_1$, $R_2$, $R_3$ et $R_4$ sont des groupes hydrocarbonés, et $R_1$ peut être relié à $R_2$, et $R_2$ peut être relié à $R_4$ pour former un cycle contenant l'atome N auquel tous deux sont reliés, et lorsque m est un nombre de 1 à 6 et n est égal à 0, $R_1$ et $R_3$ sont absents, et $R_2$ et $R_4$ sont des groupes hydrocarbonés, et lorsque m et n sont tous deux égaux à 1 au moins, $R_2$ et $R_4$ sont absents; et lorsque z est égal à 2, m et n sont égaux à 0, $R_1$ et $R_3$ sont des groupes hydrocarbonés et $R_2$ et $R_4$ sont absents, le 1,4,8,11-tétraméthyl-1,4,8,11-tétraazacyclotétradécane, la spartéine et des cryptates.

3. Procédé suivant la revendication 2, caractérisé en ce que la polyamine tertiaire linéaire est choisie parmi le 2,4-diméthyl-2,4-diazapentane, le 2,5-diméthyl-2,5-diazahexane, la 1,1'-(1,2-éthanediyl)-bis(pipéridine), le N,N,N',N'-tétraméthyl-1,2-diaminocyclohexane, le 1,4-diméthyl-1,4-diazacyclohexane, le diazabicyclo[2.2.2.]octane, le 2,6-diméthyl-2,6-diazaheptane, le 2,7-diméthyl-2,7-diazaoctane, le 2,9-diméthyl-2,9-diazadécane et le 2,5,8,11-tétraméthyl-2,5,8,11-tétraazadodécane.

4. Procédé suivant la revendication 3, caractérisé en ce que la polyamine tertiaire linéaire est le diazabicyclo[2.2.2.]-octane, le 2,6-diméthyl-2,6-diazaheptane, le 2,7-diméthyl-2,7-diazaoctane ou le 2,5,8,11-tétraméthyl-2,5,8,11-tétraazadécane.

5. Procédé suivant la revendication 4, caractérisé en ce que l'agent améliorant la vitesse est le diazabicyclo[2.2.2.]octane.

6. Procédé suivant la revendication 2, caractérisé en ce que l'agent améliorant la vitesse est un cryptate répondant à la formule:

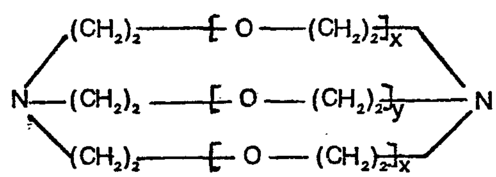

dans laquelle x et y sont égaux indépendamment à 1 ou 2.

7. Procédé suivant la revendication 6, caractérisé en ce que le cryptate est le 4,7,13,16,21,-pentaoxo-1,10-diazabicyclo-[8.8.5]tricosane.

8. Procédé suivant l'une quelconque des revendications 2 à 7, caractérisé en ce que R est un radical 3-(2,2-dichloroéthènyl)-2,2-diméthylcyclopropyle.

9. Procédé suivant l'une quelconque des revendications 2 à 7, caractérisé en ce que le solvant aprotique non miscible à l'eau est le n-heptane.

10. Procédé suivant l'une quelconque des revendications 2 à 7, caractérisé en ce que le cyanure soluble dans l'eau est le cyanure de sodium.

11. Procédé suivant la revendication 1, caractérisé en ce que l'agent améliorant la vitesse est un acide aminoalkylsulfonique choisi parmi l'acide 1,4-pipérazinebiséthanesulfonique, l'acide 4-pyridineéthanesulfonique, l'acide 2,[N,N-di-(2-hydroxy)-éthyl] aminoéthanesulfonique, l'acide 3-(cyclohexylamino) propanesulfonique et l'acide 2-[4-(2-hydroxyéthyl) pipérizine-1-yl]éthanesulfonique.

12. Procédé suivant la revendication 11, caractérisé en ce que l'agent améliorant la vitesse est choisi parmi l'acide 1,4-pipérizine-biséthanesulfonique et l'acide 3-(cyclohexylamino)propanesulfonique.

13. Procédé suivant la revendication 12, caractérisé en ce que l'agent améliorant la vitesse est l'acide 3-(cyclohexylamino)propane-sulfonique.

14. Procédé suivant l'une quelconque des revendications 11 à 13, caractérisé en ce que R est le radical 3-(2,2-dichloroéthènyl)-2,2,-diméthylcyclopropyle ou 1-(4-chlorophényl)-2-méthylpropyle.

15. Procédé suivant l'une quelconque des revendications 11 à 13, caractérisé en ce que R est un radical 3-(2,2-dichloroéthènyl)-2,2-diméthylcyclopropyle.

16. Procédé suivant l'une quelconque des revendications 11 à 13, caractérisé en ce que le solvant aprotique non miscible à l'eau est du n-heptane.

17. Procédé suivant l'une quelconque des revendications 11 à 13, caractérisé en ce que le cyanure soluble dans l'eau est du cyanure de sodium.

18. Procédé suivant la revendication 1, caractérisé en ce que l'agent améliorant la vitesse est un sel d'acide d'une amine tertiaire choisi parmi les produits de la réaction d'un acide fort et d'une polyamine tertiaire linéaire répondant à la formule:

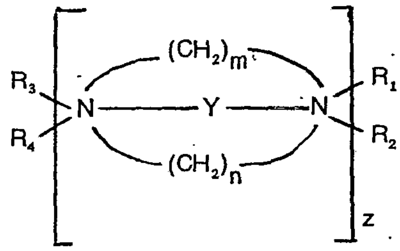

dans laquelle Y est $-(CH_2)_k-$, k étant un nombre de 1 à 6, un cycloalkane en $C_3$ à $C_7$, un alcényle en $C_2$ à $C_6$, ou un alcynyle en $C_2$ à $C_6$; z est égal à 1 ou 2 et lorsque z est égal à 1, m et n sont 0 ou sont égaux indépendamment à des nombres de 1 à 6, et lorsque m et n sont égaux à 0, $R_1$, $R_2$, $R_3$ et $R_4$ sont des groupes hydrocarbonés et $R_1$ peut être relié à $R_2$, et $R_3$ peut être relié à $R_4$ pour former un cycle contenant l'atome N auquel tous deux sont reliés, et lorsque m est un nombre de 1 á 6 et n est égal à 0, $R_1$ et $R_3$ sont absents, et $R_2$ et $R_4$ sont des groupes hydrocarbonés, et lorsque m et n sont tous deux égaux à au moins 1, $R_2$ et $R_4$ sont absents; et lorsque z est égal à 2, m et n sont égaux à 0, $R_1$ et $R_3$ sont des groupes hydrocarbonés et $R_2$ et $R_4$ sont absents.

19. Procédé suivant la revendication 18, caractérisé en ce que l'agent améliorant la vitesse est choisi parmi le dichlorhydrate de diazabicyclo[2.2.2.]octane, le dichlorhydrate de 2,7-diméthyl-2,7-diazaoctane, le chlorhydrate de 2,3,4,6,7,8,9,10-octahydropropyrimido[1,2-a]azépine et le chlorhydrate de quinuclidine.

20. Procédé suivant la revendication 19, caractérisé en ce que l'agent améliorant la vitesse est le dichlorhydrate de diazabicyclo[2.2.2.]octane.

21. Procédé suivant l'une quelconque des revendications 18 à 20, caractérisé en ce que R est un radical 3-(2,2-dichloroéthènyl)-2,2-diméthylcyclopropyle.

22. Procédé suivant l'une quelconque des revendications 18 à 20, caractérisé en ce que le solvant aproptique non miscible à l'eau est le n-heptane.

23. Procédé suivant l'une quelconque des revendications 18 à 20, caractérisé en ce que le cyanure soluble dans l'eau est le cyanure de sodium.